# EUROPÄISCHE PATENTANMELDUNG

(11) **EP 4 742 837 A1**
(43) Veröffentlichungstag der Anmeldung: **13.05.2026**
(21) Anmeldenummer: 24211204.3
(22) Anmeldetag: 06.11.2024
(51) Int. Cl.: H05G 1/12

(54) **ELEKTRONISCHE SCHALTUNG UND VERFAHREN ZUM BEREITSTELLEN EINER RÖHRENHOCHSPANNUNG FÜR EINE RÖNTGENRÖHRE, VERFAHREN ZUM BETREIBEN EINER RÖNTGENRÖHRE, RÖNTGENRÖHRENSYSTEM UND MEDIZINISCHE BILDGEBUNGSVORRICHTUNG**

(71) Anmelder: Siemens Healthineers AG, 91301 Forchheim (DE)
(72) Erfinder: Ott, Leopold, 96482 Ahorn (DE); Waffler, Stefan, 91054 Buckenhof (DE)
(74) Vertreter: Siemens Healthineers Patent Attorneys

(57) **Zusammenfassung**

Die Erfindung betrifft eine elektronische Schaltung (1) zum Bereitstellen einer Röhrenhochspannung (17) für eine Röntgenröhre (18). Die elektronische Schaltung (1) weist eine erste Wechselrichtereinheit (4) auf, dazu eingerichtet ist, eine Eingangsgleichspannung (2) zu erhalten und abhängig von einer ersten Stellgröße (10) in eine erste Wechselspannung (8) zu wandeln, sowie eine zweite Wechselrichtereinheit (5), dazu eingerichtet, die Eingangsgleichspannung (2) abhängig von einer zweiten Stellgröße (11) in eine zweite Wechselspannung (9) zu wandeln. Ein weiterer Schaltungsteil (3) ist dazu eingerichtet, abhängig von der ersten und der zweiten Wechselspannung (8, 9) die Röhrenhochspannung (17) zu erzeugen. Eine Regelanordnung (24) ist dazu eingerichtet ist, eine Regelgröße (25) zu bestimmen und die erste Stellgröße (10) und/oder die zweite Stellgröße (11) abhängig von der Regelgröße (25) zu verändern, und die Regelgröße (25) abhängig von einem ersten Wechselstrom (6) und einem zweiten Wechselstrom (7) und/oder einem ersten Gleichstrom (43) und einem zweiten Gleichstrom (44) zu bestimmen.

## Beschreibung

Die Erfindung betrifft eine elektronische Schaltung zum Bereitstellen einer Röhrenhochspannung für eine Röntgenröhre. Außerdem betrifft die Erfindung ein Röntgenröhrensystem mit einer derartigen elektronischen Schaltung und ein medizinisches Bildgebungssystem mit einem derartigen Röntgenröhrensystem. Die Erfindung betrifft darüber hinaus entsprechende Verfahren.

Eine Röntgenröhre ist eine spezielle Elektronenstrahlröhre zur Erzeugung von Röntgenstrahlung. Röntgenröhren werden in unterschiedlichen bildgebenden Verfahren verwendet und bieten eine Vielzahl von Möglichkeiten unter anderem in der modernen Medizin.

Zur Erzeugung von Röntgenstrahlung mittels einer Röntgenröhre werden freie Elektronen benötigt, die mit Hilfe einer definierten Röhrenhochspannung von einer Kathode hin zu einer Anode beschleunigt werden können. Die freigesetzten Elektronen, also Ladungen, pro Zeiteinheit, die von der Kathode zur Anode fließen, werden als Röhrenstrom bezeichnet. Die Röhrenhochspannung kann üblicherweise im Bereich von 25 kV bis 600 kV liegen. Beim Auftreffen der beschleunigten Elektronen auf die Anode geben die Elektronen Energie ab, die zu Bremsstrahlung und charakteristischer Strahlung führt. Da die auftreffenden Elektronen in alle Richtungen abgelenkt oder gestreut werden können, geben diese je nach Ablenkwinkel unterschiedlich viel Energie in Form von Bremsstrahlung ab. Es entsteht dadurch ein kontinuierliches Röntgenspektrum.

Eine Gesamteffizienz, also die Strahlenausbeute im Verhältnis zur Eingangsenergie, eines Röntgenröhrensystems kann sehr gering sein. Aufgrund dessen kann es notwendig sein, die Röntgenröhre mit einer sehr hohen Leistung zu versorgen, diese kann beispielsweise in der Größenordnung von 100 kW liegen.

Die Leistungsversorgung von Röntgenröhren erfolgt konventionell über eine leistungselektronische Wandlungskette. Diese wandelt beispielsweise zunächst eine ein- oder dreiphasige Versorgungswechselspannung in eine Eingangsgleichspannung um, welche auch als Zwischenkreisspannung bezeichnet wird. Aus dieser Zwischenkreisspannung wird in einem weiteren Schritt über eine Wechselrichterstufe eine hochfrequente (beispielsweise 30 kHz - 300 kHz) Wechselspannung mit einstellbarer Höhe erzeugt, die einen Übertragungskreis hin zur Röntgenröhre speist. Der Übertragungskreis kann dabei einen Schwingkreis, einen Hochspannungstransformator und eine hochspannungsseitige Gleichrichtung enthalten. Die hochfrequente Wechselspannung kann in ihrer Höhe zum Beispiel eingestellt werden indem die Zwischenkreisspannung aktiv geregelt wird, indem wenigstens ein Brückenzweig eines Wechselrichters phasenverschoben angesteuert wird oder indem eine Frequenz der Wechselspannung variiert wird.

Das Ziel dieses Übertragungskreises ist es, eine Hochspannungsgleichspannung zwischen Anode und Kathode innerhalb der Röhre zu erzeugen, die für eine Beschleunigung der freien Elektronen aus einem entsprechend geheizten Emitter, der auch als Filament bezeichnet werden kann, auf einer Seite der Kathode sorgt und somit den Strom innerhalb der Röntgenröhre formt. Dieser Stromfluss erzeugt wiederum beim Auftreffen auf die Anode hochenergetische Röntgenstrahlung, die für die medizinische Bildgebung verwendet werden kann.

Da Röntgenröhren, insbesondere für CT- und Radiographieanwendungen, maximale Strahlleistungen im Bereich von 100 kW und darüber aufweisen können, kann beispielsweise aus Gründen der thermischen Belastung der Bauteile der Wechselrichterstufe die Leistungsübertragung auf mehrere Stufen aufgeteilt werden. Dadurch kann es durch eine unweigerlich vorhandene Toleranz in verschiedenen Stufen, beispielsweise einer Toleranz von Bauteilen des Übertragungskreises, zu einer asymmetrischen Strom- und damit Leistungsaufteilung kommen, was nachteilhaft ist.

Es ist eine Aufgabe der vorliegenden Erfindung bei mehrstufigen Versorgungsschaltungen für die Röhrenhochspannung einer Röntgenröhre die Asymmetrie der Lastverteilung zu reduzieren.

Diese Aufgabe wird durch den jeweiligen Gegenstand der unabhängigen Ansprüche gelöst. Vorteilhafte Weiterbildungen und bevorzugte Ausführungsformen sind Gegenstand der abhängigen Ansprüche, der folgenden Beschreibung sowie der Figuren.

Die Erfindung beruht auf dem Gedanken durch eine ausgangsseitige Regelung im Rahmen der Wechselspannungserzeugung eine symmetrischere Lastverteilung der mehrstufigen Versorgungsschaltung sicherzustellen.

Gemäß einem Aspekt der Erfindung wird eine elektronische Schaltung zum Bereitstellen einer Röhrenhochspannung für eine Röntgenröhre vorgestellt. Die elektronische Schaltung weist eine erste Wechselrichtereinheit auf, die dazu eingerichtet ist, eine Eingangsgleichspannung zu erhalten und die Eingangsgleichspannung abhängig von einer ersten Stellgröße in eine erste Wechselspannung zu wandeln. Außerdem weist die elektronische Schaltung eine zweite Wechselrichtereinheit auf, die dazu eingerichtet ist, die Eingangsgleichspannung zu erhalten und die Eingangsgleichspannung abhängig von einer zweiten Stellgröße in eine zweite Wechselspannung zu wandeln. Des Weiteren weist die elektronische Schaltung einen weiteren Schaltungsteil auf, der dazu eingerichtet ist, abhängig von der ersten Wechselspannung und der zweiten Wechselspannung die Röhrenhochspannung zu erzeugen und die Röhrenhochspannung ausgangsseitig, also an einem Ausgang des Weiteren Schaltungsteils, zur Verfügung zu stellen. Außerdem weist die elektronische Schaltung eine Regelanordnung auf, die dazu eingerichtet ist, eine Regelgröße zu bestimmen und die erste Stellgröße und/oder die zweite Stellgröße abhängig von der Regelgröße zu verändern, um die Regelgröße auf einen vorgegebenen Sollwert zu regeln. Dabei ist die Regelanordnung dazu eingerichtet, die Regelgröße zu bestimmen abhängig von
i) einem aus der ersten Wechselspannung resultierenden ersten Wechselstrom und einem aus der zweiten Wechselspannung resultierenden zweiten Wechselstrom und/oder
ii) einem aus der Eingangsgleichspannung resultierenden ersten Gleichstrom an der ersten Wechselrichtereinheit und einem aus der Eingangsgleichspannung resultierenden zweiten Gleichstrom an der zweiten Wechselrichtereinheit.

Die Regelgröße muss dabei nicht notwendigerweise direkt aus dem ersten Wechselstrom und dem zweiten Wechselstrom und/oder dem ersten Gleichstrom und dem zweiten Gleichstrom bestimmt werden, sondern kann auch aus anderen physikalischen Größen bestimmt werden, die von den diesen Wechselströmen und/oder diesen Gleichströmen abhängen.

Die elektronische Schaltung kann insbesondere mindestens zwei Ausgangsanschlüsse enthalten, die dazu ausgelegt sind, die Röhrenhochspannung an die Röntgenröhre bereitzustellen. Die Röhrenhochspannung kann insbesondere zwischen einer Anode und eine Kathode der Röntgenröhre angelegt werden, um Röntgenstrahlung zu erzeugen. Bei der Röhrenhochspannung kann es sich insbesondere um eine Gleichspannung handeln. Für diesen Fall kann ein erster Ausgangsanschluss ein positives elektrisches Potential aufweisen, das an der Anode der Röntgenröhre angelegt werden kann und ein zweiter Ausgangsanschluss ein negatives elektrisches Potential aufweisen, das an der Kathode der Röntgenröhre angelegt werden kann.

Die erste Wechselrichtereinheit kann einen ersten Wechselrichter enthalten oder aus einem ersten Wechselrichter bestehen und die zweite Wechselrichtereinheit kann einen zweiten Wechselrichter enthalten oder aus einem zweiten Wechselrichter bestehen. Die erste Stellgröße der ersten Wechselrichtereinheit kann insbesondere ein erster Aussteuergrad des ersten Wechselrichters sein oder ein anderer Parameter des ersten Wechselrichters, der die erste Wechselspannung, insbesondere deren Effektivwert, beeinflusst. Die zweite Stellgröße der zweiten Wechselrichtereinheit kann insbesondere ein zweiter Aussteuergrad des zweiten Wechselrichters sein oder ein anderer Parameter des zweiten Wechselrichters, der die zweite Wechselspannung, insbesondere deren Effektivwert, beeinflusst. Der jeweilige Wechselrichter kann insbesondere eine Vollbrücke oder eine Halbbrücke in Kombination mit einem Spannungsteiler, beispielsweise einem kapazitiven Spannungsteiler, welcher die Eingangsgleichspannung halbiert, aufweisen. Die Vollbrücke oder die Halbbrücke können zum Beispiel über ein PWM Signal angesteuert werden.

Insbesondere kann eine erste Amplitude der ersten Wechselspannung von dem ersten Aussteuergrad abhängen und eine zweite Amplitude der zweiten Wechselspannung von dem zweiten Aussteuergrad abhängen. Eine erste Schaltfrequenz des ersten Wechselrichters und eine zweite Schaltfrequenz des zweiten Wechselrichters sind insbesondere gleich, um einen Schwebungseffekt zwischen dem ersten Wechselrichter und dem zweiten Wechselrichter zu vermeiden.

Ein resultierender Wechselstrom kann hier und im Folgenden insbesondere gemäß dem ohmschen Gesetz als ein durch eine Last hervorgerufener Wechselstrom sein, der entsteht, sobald eine entsprechende Wechselspannung mit der Last verbunden wird. Dies gilt insbesondere sowohl für den ersten Wechselstrom, als auch für den zweiten Wechselstrom, als auch für alle weiteren Wechselströme.

Ein resultierender Gleichstrom kann hier und im Folgenden insbesondere gemäß dem ohmschen Gesetz als ein durch eine weitere Last hervorgerufener Gleichstrom sein, der entsteht, sobald eine entsprechende Gleichspannung mit der weiteren Last verbunden wird. Dies gilt insbesondere sowohl für den ersten Gleichstrom, als auch für den zweiten Gleichstrom, als auch für alle weiteren Gleichströme.

Die Regelanordnung enthält beispielsweise eine Messeinrichtung, die dazu eingerichtet ist, sowohl den ersten Wechselstrom als auch den zweiten Wechselstrom ausgangsseitig des jeweiligen Wechselrichters zu messen und/oder den ersten Gleichstrom und den zweiten Gleichstrom eingangsseitig des jeweiligen Wechselrichters zu messen. Beispielsweise enthält die Regelanordnung ein Strommessgerät. Dabei können die jeweiligen Werte unabhängig voneinander gemessen werden.

Die Regelanordnung kann ebenso beispielsweise eine Schaltung zur Bestimmung eines ersten charakteristischen Werts des ersten Wechselstroms und eines zweiten charakteristischen Werts des zweiten Wechselstroms enthalten. Der erste charakteristische Wert kann beispielsweise einem ersten Effektivwert, einem ersten Gleichrichtwert oder einem ersten Spitzenwert entsprechen. Der zweite charakteristische Wert kann beispielsweise einem zweiten Effektivwert, einem zweiten Gleichrichtwert oder einem zweiten Spitzenwert entsprechen.

Die Werte für einen Gleichrichtwert und/oder für einen Spitzenwert können hier und im Folgenden jeweils innerhalb einer Schaltperiode ermittelt werden.

Ebenso enthält die Regelanordnung beispielsweise einen Regler, der dazu eingerichtet ist, die Regelgröße zu bestimmen und abhängig von der Regelgröße und dem vorgegebenen Sollwert die erste Stellgröße und die zweite Stellgröße auszugeben. Die Regelgröße kann sich aus den gemessenen Werten des ersten Wechselstroms, des ersten Gleichstroms oder des ersten charakteristischen Werts und des zweiten Wechselstroms, des zweiten Gleichstroms oder des zweiten charakteristischen Werts zusammensetzen beziehungsweise abhängig von diesen bestimmt werden, also abhängig nur von dem ersten Wechselstrom oder dem ersten Gleichstrom und dem zweiten Wechselstrom oder dem zweiten Gleichstrom oder abhängig zusätzlich von weiteren Größen. Die Bestimmung der Regelgröße kann insbesondere eine Differenzbildung des ersten charakteristischen Werts und des zweiten charakteristischen Werts beinhalten.

Der vorgegebene Sollwert kann im Falle der Differenzbildung bei der Ermittlung der Regelgröße insbesondere gleich null sein. In diesem Fall kann die Regelung auch als Nullwertregelung bezeichnet werden. Die erste Stellgröße und/oder die zweite Stellgröße werden dann beispielsweise so lange verändert, solange der erste charakteristischen Wert ungleich dem zweiten charakteristischen Wert ist. Dabei kann ein Wert insbesondere auch als gleich angesehen werden, wenn er unter realen Bedingungen eine toleranzbehaftete Abweichung aufweist. Die Abweichung kann im Bereich von wenigen Prozent des ersten charakteristischen Werts oder des zweiten charakteristischen Werts liegen, insbesondere im Bereich von kleiner 5%.

Ebenso kann der Regler innerhalb eines Regelprozesses ein Hystereseverhalten aufweisen. Darunter ist eine Regelung zu verstehen, die nicht nur vom aktuellen Wert der Regelgröße abhängt, sondern auch vom vorherigen. Damit kann sich die Regelung unterschiedlich verhalten, je nachdem, ob die Regelung von einem höheren Wert startet und sich auf einen niedrigeren Wert zubewegt oder umgekehrt.

Ebenso kann auf abweichende vorgegebene Sollwerte geregelt werden.

Bei der vorgestellten Erfindung kann die Regelung eine Veränderung nur der ersten Stellgröße und nicht der zweiten Stellgröße oder eine Veränderung nur der zweiten Stellgröße und nicht der ersten Stellgröße enthalten. Dies kann insbesondere notwendig oder gewollt sein, wenn nur eine Anpassung oder Veränderung der ersten Wechselrichtereinheit beziehungsweise nur eine Anpassung oder Veränderung der zweiten Wechselrichtereinheit hervorgerufen werden soll und die jeweils andere Wechselrichtereinheit unverändert betrieben werden soll. In anderen Ausführungsformen kann es günstig sein, beide Stellgrößen innerhalb der Regelung zu verändern und so möglicherweise einen Regelablauf zu beschleunigen.

Ein Vorteil der beschriebenen Erfindung ist, dass eine mehrstufige Versorgungsschaltung für die Röhrenhochspannung symmetrisch betrieben werden kann, ohne bei der Zusammenführung der Spannungen zusätzliche Bauteile zu verwenden. Ein symmetrischer Betrieb kann beispielsweise Vorteile bei einer Wärmeverteilung und einer Lebensdauer der elektronischen Schaltung hervorrufen.

Die Zusammenführung der jeweiligen Spannungspotentiale kann durch einfache Knotenpunkte, also eine direkte Verbindung ausgeführt werden, ohne dass eine ungleichmäßige Belastung eines Versorgungszugs zu erwarten ist. Eine solche ungleichmäßige Belastung ist in bekannten mehrstufigen Versorgungsschaltungen durch Bauteiltoleranzen unvermeidbar. Durch die erfindungsgemäße elektronische Schaltung werden die Wechselströme oder die Gleichströme insbesondere aktiv ermittelt und die Regelgröße abhängig davon bestimmt. Die erfindungsgemäße elektronische Schaltung ist also nicht oder weniger stark von vorher bekannten oder selektierten Bauteiltoleranzen abhängig und die Regelung kann sich auf die jeweils vorliegende Konstellation anpassen. Ein weiterer Vorteil ist also, dass Bauteile der elektronischen Schaltung unabhängig voneinander innerhalb der industrieüblichen Bauteiltoleranzen verbaut werden können.

Gemäß zumindest einer Ausführungsform der elektronischen Schaltung enthält die Regelanordnung einen PI Regler oder einen PID Regler.

Der PI Regler oder der PID Regler ist insbesondere dazu eingerichtet, die erste Stellgröße und/oder die zweite Stellgröße abhängig von der Regelgröße zu verändern.

Der PI Regler (proportional-integral controller) enthält sowohl eine proportionale Komponente als auch eine integrale Komponente. Bei der proportionalen Komponente ist der Zusammenhang zwischen einer Eingangs- und einer Ausgangsgröße definiert durch eine Sprungfunktion mit einer festgelegten Verstärkung. Die integrale Komponente zeigt zwischen Eingang und Ausgang einen linear ansteigenden zeitlichen Verlauf. Der PID Regler (proportional- integral-derivative controller) enthält zusätzlich eine differentielle Komponente. Eine Sprungantwort ist eine Stoßfunktion mit theoretisch unendlicher Größe.

Gemäß zumindest einer Ausführungsform der elektronischen Schaltung hängt die Regelgröße von einer Differenz zwischen dem ersten Wechselstrom und dem zweiten Wechselstrom oder einer Differenz zwischen dem ersten Gleichstrom und dem zweiten Gleichstrom ab.

Mit anderen Worten wird ein Wert des ersten Wechselstroms oder des ersten Gleichstroms von einem Wert des zweiten Wechselstroms oder des zweiten Gleichstroms abgezogen oder umgekehrt. Im Fall von Wechselströmen kann dies zum Beispiel ein charakteristischer Wert des ersten Wechselstroms oder ein charakteristischer Wert des zweiten Wechselstroms sein. Ebenso kann es dabei möglich sein, dass der erste Wechselstrom oder der erste Gleichstrom zunächst eine erste Filtereinheit durchläuft und der zweite Wechselstrom oder der zweite Gleichstrom zunächst eine zweite Filtereinheit durchläuft. Im Anschluss kann die Differenz der beiden Ausgangswerte der beiden Filtereinheiten gebildet werden. Insbesondere können sowohl die erste Filtereinheit als auch die zweite Filtereinheit jeweils einen RMS-Filter (root mean square Filter) aufweisen.

Zum Beispiel kann im Fall, dass der erste Wechselstrom oder der erste Gleichstrom ein Minuend ist und der zweite Wechselstrom oder der zweite Gleichstrom ein Subtrahend ist, die erste Stellgröße nach unten, das heißt zu einem kleineren Wert hin, verändert werden und die zweite Stellgröße nach oben, das heißt zu einem größeren Wert hin verändert werden. Insbesondere ist es auch möglich, dass die Regelgröße abhängig ist von einem Betrag der Differenz zwischen dem ersten Wechselstrom oder dem ersten Gleichstrom und dem zweiten Wechselstrom oder dem zweiten Gleichstrom. Ebenso ist es möglich, dass die Regelgröße von einem vordefinierten Vielfachen oder von einem vordefinierten Bruchteil der Differenz zwischen dem ersten Wechselstrom oder dem ersten Gleichstrom und dem zweiten Wechselstrom oder dem zweiten Gleichstrom abhängig ist.

Die Regelgröße ist in den dargestellten Ausführungsformen abhängig von der Differenz der Wechselströme oder der Differenz der Gleichströme, die ausschlaggebend für die Belastung des jeweiligen Versorgungszugs sind. Insbesondere kann der vorgegebene Sollwert null sein, sodass die Regelung das Ziel haben kann, die Wechselströme oder die Gleichströme aneinander anzupassen. Eine solche Regelung kann ebenso als Nullwertregelung bezeichnet werden.

Ein Vorteil dieser Ausführungsform ist der einfache Aufbau und die Möglichkeit der Regelung auf einen Nullwert, also eine Anpassung der beiden Wechselströme oder der beiden Gleichströme auf den gleichen Wert, insbesondere auf den gleichen Effektivwert der beiden Wechselströme.

Gemäß zumindest einer weiteren Ausführungsform der elektronischen Schaltung ist die Regelanordnung dazu eingerichtet, aus dem ersten Wechselstrom einen ersten charakteristischen Wert zu bestimmen und aus dem zweiten Wechselstrom einen zweiten charakteristischen Wert zu bestimmen. Außerdem ist die Regelgröße abhängig von dem ersten charakteristischen Wert und dem zweiten charakteristischen Wert oder die Regelgröße ist abhängig ist von einer Differenz zwischen dem ersten charakteristischen Wert und dem zweiten charakteristischen Wert. Der erste charakteristische Wert entspricht einem ersten Effektivwert, einem ersten Gleichrichtwert oder einem ersten Spitzenwert und der zweite charakteristische Wert entspricht einem zweiten Effektivwert, einem zweiten Gleichrichtwert oder einem zweiten Spitzenwert.

Es kann dabei möglich sein, dass der erste Wechselstrom zunächst die erste Filtereinheit durchläuft und der zweite Wechselstrom zunächst die zweite Filtereinheit durchläuft. Im Anschluss kann die Differenz der beiden Ausgangswerte der beiden Filtereinheiten gebildet werden. Insbesondere können sowohl die erste Filtereinheit als auch die zweite Filtereinheit jeweils einen RMS-Filter (root mean square Filter) aufweisen.

Zum Beispiel kann im Fall, dass der erste charakteristischen Wert der Minuend ist und der zweite charakteristischen Wert der Subtrahend, die erste Stellgröße nach unten, das heißt zu einem kleineren Wert hin verändert werden und die zweite Stellgröße nach oben, das heißt zu einem größeren Wert hin verändert werden. Insbesondere ist es auch möglich, dass die Regelgröße abhängig ist von einem Betrag der Differenz zwischen dem ersten charakteristischen Wert und dem zweiten charakteristischen Wert.

Die Regelgröße ist in den dargestellten Ausführungsformen abhängig von der Differenz der charakteristischen Werte, die ausschlaggebend für die Belastung des jeweiligen Versorgungszugs sind. Insbesondere kann der vorgegebene Sollwert null sein, sodass die Regelung das Ziel haben kann, die charakteristischen Werte aneinander anzupassen. Eine solche Regelung kann ebenso als Nullwertregelung bezeichnet werden.

Ein Vorteil dieser Ausführungsform ist der einfache Aufbau und eine einfache Weiterverarbeitung der charakteristischen Werte innerhalb der Regelung. Es können diverse Operationen mit den vorliegenden charakteristischen Werten durchgeführt werden, insbesondere im Fall von zwei Versorgungszügen eine Differenz.

Gemäß zumindest einer weiteren Ausführungsform der elektronischen Schaltung ist die Regelanordnung dazu eingerichtet, abhängig von dem ersten charakteristischen Wert und dem zweiten charakteristischen Wert einen größten charakteristischen Wert, also ein Maximum des ersten charakteristischen Werts und des zweiten charakteristischen Werts, zu ermitteln. Außerdem ist die Regelanordnung dazu eingerichtet, die erste Stellgröße abhängig von der Regelgröße zu verändern, wenn der erste charakteristische Wert geringer als der größte charakteristische Wert ist, und die zweite Stellgröße abhängig von der Regelgröße zu verändern, wenn der zweite charakteristische Wert geringer als der größte charakteristische Wert ist.

Als charakteristischer Wert eines Wechselstroms kann ein Wert eines vergleichbaren Gleichstroms verstanden werden, der in einem ohmschen Widerstand dieselbe Wärme erzeugt, beispielsweise ein Effektivwert des Wechselstroms. Der Effektivwert kann mit einem effektivwertbildenden Strommessgerät gemessen werden. Zur Ermittlung des größten charakteristischen Werts kann der erste charakteristische Wert mit dem zweiten charakteristischen Wert verglichen werden und der größte charakteristische Wert kann einem größten Wert der beiden entsprechen.

Hier und im Folgenden kann als charakteristischer Wert eines Gleichstroms insbesondere auch der Wert des Gleichstroms angenommen werden. Insbesondere kann die Regelanordnung auch dazu ausgebildet sein, den erste charakteristische Wert abhängig von dem ersten Gleichstrom und den zweiten charakteristischen Wert abhängig von dem zweiten Gleichstrom zu bestimmen.

Mit anderen Worten kann in der Ausführungsform die entsprechende Stellgröße derjenigen Wechselrichtereinheit, die den größten charakteristischen Wert liefert, unverändert bleiben. Damit kann der Wechselrichter dieser Wechselrichtereinheit eine unveränderte Wechselspannung liefern. Außerdem kann die entsprechende Stellgröße der jeweils anderen Wechselrichtereinheit angepasst werden, sodass sich die Wechselspannung, die der Wechselrichter dieser Wechselrichtereinheit liefert, verändern kann.

Es kann insbesondere gewollt sein, die Stellgröße, die auch als Aussteuergrad der ersten bzw. zweiten Wechselrichtereinheit ausgeführt sein kann, initial auf einen möglichst geringen Wert zu setzen. Wenn der charakteristische Wert der ersten bzw. der zweiten Wechselrichtereinheit geringer ist, als der größte charakteristische Wert, kann die Stellgröße dieser Wechselrichtereinheit dann zum Beispiel nur nach oben, also zu höheren Werten hin, angepasst werden, da eine Anpassung nach unten, also zu geringeren Werten hin, systemseitig nicht mehr möglich ist.

Ein Vorteil dieser Ausführungsform ist der einfache Aufbau und die Stabilität des gelieferten Wechselstroms der Wechselrichtereinheit, die den höheren Wechselstrom liefert oder mit dem höheren Gleichstrom versorgt wird. Diese Wechselrichtereinheit wird nicht angepasst und dies kann zu einer Stabilisierung der Röhrenhochspannung beitragen.

Gemäß zumindest einer weiteren Ausführungsform der elektronischen Schaltung ist die Regelanordnung dazu eingerichtet, abhängig von der Regelgröße und dem vorgegebenen Sollwert eine Reglerstellgröße zu ermitteln. Außerdem hängt die erste Stellgröße von einer Differenz eines vorgegebenen Initialwerts und der Reglerstellgröße ab und/oder hängt die zweite Stellgröße von einer Summe des vorgegebenen Initialwerts und der Reglerstellgröße ab.

Mit anderen Worten kann sowohl die erste Wechselrichtereinheit als auch die zweite Wechselrichtereinheit mit dem vorgegebenen Initialwert initialisiert werden. Das heißt, wenn die jeweilige Stellgröße ein Aussteuergrad ist, können die Wechselrichter der jeweiligen Wechselrichtereinheiten mit dem gleichen Aussteuergrad initialisiert werden. Der vorgegebene Initialwert kann ein Ergebnis eines vorgelagerten Rechenprozesses sein, der zum Beispiel abhängig ist von vorangegangenen Simulationen oder Messungen.

Anschließend kann innerhalb des Regelprozesses die Reglerstellgröße ermittelt werden. Die Reglerstellgröße kann ein Ausgangssignal des Reglers sein und abhängig sein von der Regelgröße und dem vorgegebenen Sollwert. Die Reglerstellgröße kann von der ersten Stellgröße abgezogen werden, das heißt der erste Wechselrichter kann ein geringeres Signal erhalten. Ebenso kann die Reglerstellgröße zu der zweiten Stellgröße hinzugerechnet werden, das heißt der zweite Wechselrichter kann ein größeres Signal erhalten. Ebenso kann diese Vorgehensweise auf eine größere Anzahl an Wechselrichtereinheiten ausgeweitet werden.

Ebenso kann die Regelanordnung einen Pl Regler oder einen PID Regler enthalten. Der Pl Regler oder der PID Regler kann insbesondere dazu eingerichtet sein, die Reglerstellgröße abhängig von der Regelgröße zu verändern.

Ein Vorteil dieser Ausführungsform ist die gleichmäßige Initialisierung der Wechselrichtereinheiten und damit die schnellere Regelung auf einen gewollten Wert der Röhrenhochspannung.

Gemäß zumindest einer weiteren Ausführungsform enthält der weitere Schaltungsteil einen Spannungstransformator, der dazu eingerichtet ist, primärseitig eine aus der ersten Wechselspannung und/oder der zweiten Wechselspannung resultierende Primärwechselspannung zu erhalten und die Primärwechselspannung in eine Sekundärwechselspannung zu wandeln. Außerdem ist die Röhrenhochspannung abhängig von der Sekundärwechselspannung.

Insbesondere für den Fall, dass der weitere Schaltungsteil genau einen Spannungstransformator enthält, wird primärseitig des Spannungstransformators eine Kopplung der ersten Wechselspannung und der zweiten Wechselspannung mit zum Beispiel zwei Anschlüssen benötigt. Diese Kopplung kann zum Beispiel durch zwei direkte leitfähige Verbindungen, also zwei Knotenpunkte ausgeführt sein. Insbesondere im Fall, dass der weitere Schaltungsteil den oben beschriebenen ersten Resonanzkreis und/oder den zweiten Resonanzkreis enthält, kann die ausgangsseitig des jeweiligen Resonanzkreises zur Verfügung gestellte Wechselspannung in dem Knotenpunkt auf ein identisches Spannungspotential, nämlich das Spannungspotential der Primärwechselspannung, gezwungen werden und dieses primärseitig am Spannungstransformator anliegen. Der Spannungstransformator kann dabei ebenso aufgrund des Übersetzungsverhältnisses sekundärseitig eine höhere, insbesondere eine deutlich höhere, Spannungsamplitude ausgeben, als er primärseitig erhält.

Ebenso ist es möglich, dass der weitere Schaltungssteil einen weiteren Spannungstransformator enthält. In diesem Fall kann die erste Wechselspannung zum Beispiel primärseitig am Spannungstransformator anliegen, entweder direkt oder über den ersten Resonanzkreis. Ebenso kann die zweite Wechselspannung entweder direkt oder über den zweiten Resonanzkreis an dem weiteren Spannungstransformator anliegen. Dann kann die jeweilige Sekundärwechselspannung zum Beispiel durch zwei weitere Knotenpunkte miteinander verbunden werden.

Ein Vorteil dieser Ausführungsform ist die galvanische Trennung und die Erhöhung der Spannungsamplitude durch den Spannungstransformator.

Gemäß einer weiteren Ausführungsform enthält der weitere Schaltungsteil einen Gleichrichter, insbesondere genau einen Gleichrichter, der dazu eingerichtet ist, die Sekundärwechselspannung in die Röhrenhochspannung zu wandeln.

Der Gleichrichter kann die Röhrenhochspannung zum Beispiel an zwei Anschlusspunkten der Röntgenröhre zur Verfügung stellen. Die beiden Anschlusspunkte des Gleichrichters können insbesondere identisch sein mit den Ausgangsanschlüssen der elektronischen Schaltung.

Ebenso kann der Gleichrichter eine alternative Wechselspannung, die abhängig ist von der ersten Wechselspannung und der zweiten Wechselspannung erhalten, insbesondere durch eine Verbindung der beiden Wechselspannungen durch einen alternativen Knotenpunkt. Der Gleichrichter kann ebenso die alternative Wechselspannung in die Röhrenhochspannung wandeln und diese der Röntgenröhre zur Verfügung stellen.

Ebenso kann der weitere Schaltungsteil einen weiteren Gleichrichter enthalten. In diesem Fall kann die jeweilige ausgangsseitige Röhrenhochspannung mit einem zusätzlichen Knotenpunkt miteinander gekoppelt sein.

Unter einem Knotenpunkt, an dem Spannungen miteinander gekoppelt sind, kann hier und im Folgenden verstanden werden, dass an diesem Punkt eine leitende Verbindung mit einem geringen ohmschen Widerstand besteht, insbesondere mit einem ohmschen Widerstand von null, und dadurch im Betrieb das elektrische Potential auf einen identischen Wert im Knotenpunkt gezwungen wird.

Ein Vorteil dieser Ausführungsform ist, dass der Gleichrichter die Röhrenhochspannung in einer Form zur Verfügung stellt, die in der Röntgenröhre zur Erzeugung von Röntgenstrahlung verwendet werden kann.

Gemäß einer weiteren Ausführungsform enthält der weitere Schaltungsteil einen ersten Resonanzkreis, der eingangsseitig, also an einem Eingang des Resonanzkreises, mit einem Ausgang der ersten Wechselrichtereinheit und ausgangsseitig, also an einem Ausgang des weiteren Resonanzkreises, mit einem Ausgang des Weiteren Schaltungsteils, der zum Bereitstellen der Röhrenhochspannung eingerichtet ist, gekoppelt ist. Außerdem enthält der weitere Schaltungsteil einen zweiten Resonanzkreis, der eingangsseitig mit einem Ausgang der zweiten Wechselrichtereinheit und ausgangsseitig mit dem Ausgang des Weiteren Schaltungsteils, der zum Bereitstellen der Röhrenhochspannung eingerichtet ist, gekoppelt ist.

Der erste Resonanzkreis und der zweite Resonanzkreis können beispielsweise jeweils aus einer Serienschaltung bestehend aus einer Kapazität und einer Induktivität aufgebaut sein. Ebenso können im ersten Resonanzkreis und im zweiten Resonanzkreis weitere Bauteile in Serie und/oder parallel enthalten sein.

Ein Vorteil dieser Ausführungsform ist eine mögliche Kompensation von parasitären Elemente der übrigen elektronischen Schaltung und damit eine geringere Blindleistung, die durch einen Aufbau oder einen Abbau von elektrischen und magnetischen Feldern in kapazitiven oder induktiven Elementen auftauchen kann.

Gemäß einer weiteren Ausführungsform ist ein Ausgang des ersten Resonanzkreises mit einem Ausgang des zweiten Resonanzkreises gekoppelt.

Insbesondere können ein Ausgang des ersten Resonanzkreises bestehend aus zwei Klemmenanschlüssen und ein Ausgang des zweiten Resonanzkreises bestehend aus zwei weiteren Klemmenanschlüssen miteinander verbunden sein, das heißt beispielsweise kurzgeschlossen sein. Dadurch können die beiden Resonanzkreise zum Beispiel mit einem Eingang des Spannungstransformators verbunden sein.

Alternativ kann eine Ausführungsform auch den weiteren Spannungstransformator enthalten. Dann kann der Ausgang des ersten Resonanzkreises mit dem Spannungstransformator verbunden sein und der Ausgang des zweiten Resonanzkreises mit dem weiteren Spannungstransformator verbunden sein.

Ein Vorteil dieser Ausführungsform ist eine Leistungszusammenführung der enthaltenen Versorgungszüge und damit eine Erhöhung einer Gesamtleistung, die an der Röntgenröhre zur Verfügung steht. Der erste und der zweite Resonanzkreis können entsprechend ausgelegt werden, um einen Spannungs- und einen Stromverlauf zu optimieren.

Gemäß einer weiteren Ausführungsform enthält die elektronische Schaltung wenigstens eine weitere Wechselrichtereinheit, wobei jede der wenigstens einen weiteren Wechselrichtereinheiten dazu eingerichtet ist, die Eingangsgleichspannung zu erhalten und die Eingangsgleichspannung abhängig von einer jeweiligen weiteren Stellgröße in eine jeweilige weitere Wechselspannung zu wandeln. Außerdem ist der weitere Schaltungsteil dazu eingerichtet, die Röhrenhochspannung abhängig von den weiteren Wechselspannungen, also insbesondere abhängig von allen mittels der wenigstens einen weiteren Wechselrichtereinheit erzeugten weiteren Wechselspannungen, zu erzeugen. Des Weiteren ist die Regelanordnung dazu eingerichtet, die Regelgröße zu bestimmen abhängig von
i) dem ersten Wechselstrom, dem zweiten Wechselstrom und einem jeweiligen aus den weiteren Wechselspannungen resultierenden weiteren Wechselstrom und/oder
ii) dem ersten Gleichstrom, dem zweiten Gleichstrom und einem jeweiligen aus der Eingangsgleichspannung resultierenden weiteren Gleichstrom an jeder weiteren Wechselrichtereinheit der wenigstens einen weiteren Wechselrichtereinheit.

In manchen Ausführungsformen ist die Regelanordnung dazu eingerichtet, die jeweilige weitere Stellgröße abhängig von der Regelgröße zu verändern, um die Regelgröße auf den vorgegebenen Sollwert zu regeln.

Insbesondere lassen sich die weiter oben beschriebenen Ausführungsformen ebenso durch die wenigstens eine weitere Wechselrichtereinheit erweitern. Beispielhaft sei im Folgenden eine Ausführungsform mit drei Wechselrichtereinheiten dargelegt, das heißt also, dass die wenigstens eine weitere Wechselrichtereinheit genau eine weitere Wechselrichtereinheit ist. Jede der drei Wechselrichtereinheiten, also die erste, die zweite und die weitere Wechselrichtereinheit kann eingangsseitig die Eingangsspannung erhalten. Ebenso kann jede der drei Wechselrichtereinheiten von einer individuellen Stellgröße abhängig arbeiten, die erste Wechselrichtereinheit von der ersten Stellgröße, die zweite Wechselrichtereinheit von der zweiten Stellgröße und die weitere Wechselrichtereinheit von einer weiteren Stellgröße. Der weitere Schaltungsteil kann dazu eingerichtet sein, die Röhrenhochspannung abhängig von der ersten Wechselspannung, der zweiten Wechselspannung und der weiteren Wechselspannung zu erzeugen. Jeder der in diesem Beispiel beschriebenen drei Wechselrichtereinheiten kann einen aus der jeweiligen Wechselspannung resultierenden Wechselstrom hervorrufen, die erste Wechselrichtereinheit den ersten Wechselstrom, die zweite Wechselrichtereinheit den zweiten Wechselstrom und die weitere Wechselrichtereinheit einen weiteren Wechselstrom. Ebenso kann jede der drei Wechselrichtereinheiten einen aus der Eingangsgleichspannung resultierenden Gleichstrom hervorrufen, die erste Wechselrichtereinheit den ersten Gleichstrom, die zweite Wechselrichtereinheit den zweiten Gleichstrom und die weitere Wechselrichtereinheit einen weiteren Gleichstrom. Die Regelanordnung kann dazu eingerichtet sein, aus dem ersten Wechselstrom, dem zweiten Wechselstrom und dem weiteren Wechselstrom oder aus dem ersten Gleichstrom, dem zweiten Gleichstrom und dem weiteren Gleichstrom, die Regelgröße zu bestimmen. Die Regelanordnung kann abhängig von der Regelgröße die erste Stellgröße und/oder die zweite Stellgröße und/oder die weitere Stellgröße verändern. Es können also sowohl alle drei Stellgrö-ßen verändert werden, das heißt alle drei Wechselrichtereinheiten beeinflusst werden, oder zwei der drei Wechselrichtereinheiten oder lediglich eine der drei Wechselrichtereinheiten.

Insbesondere kann die Regelanordnung auch dazu eingerichtet sein, zunächst diejenige Wechselrichtereinheit zu ermitteln, die den größten Wechselstrom oder den größten Gleichstrom liefert. Diese Wechselrichtereinheit kann im Regelprozess zum Beispiel unverändert gelassen werden. Im Anschluss kann eine Differenz der beiden kleineren Wechselströme oder Gleichströme der jeweils anderen beiden Wechselrichtereinheiten ermittelt werden. Die Regelgröße kann dann abhängig von dieser Differenz sein oder gleich dieser Differenz sein. Daraufhin können die beiden Stellgrößen der beiden Wechselrichtereinheiten abhängig von der Regelgröße verändert werden.

Das beschriebene Beispiel lässt sich auch auf mehrere weitere Wechselrichtereinheiten ausweiten, wobei stets ein der jeweiligen Wechselrichtereinheit eigener durch die jeweilige Wechselspannung resultierender Wechselstrom oder ein der jeweiligen Wechselrichtereinheit eigener durch die Eingangsgleichspannung resultierender Gleichstrom als abhängige Größe in die Regelgröße einfließen kann und damit den Regelprozess verändern kann. Insbesondere kann die Regelgröße abhängig sein von allen resultierenden Wechselströmen und/oder allen Gleichströmen aus den Wechselrichtereinheiten.

Ein Vorteil der beschriebenen Ausführungsform ist die Skalierbarkeit der vorgestellten Erfindung. Je nach Leistungsbedarf kann eine weitere Leistungsstufe hinzugefügt werden und der Regelprozess entsprechend erweitert werden.

Gemäß einem weiteren Aspekt der Erfindung wird ein Röntgenröhrensystem, insbesondere ein Röntgenröhrensystem für ein medizinisches Bildgebungssystem, angegeben, aufweisend die erfindungsgemäße elektronische Schaltung und die Röntgenröhre.

Die Röntgenröhre kann dabei eine Kathode und eine Anode zur Erzeugung eines Röntgenstroms enthalten, die durch die elektronische Schaltung mit einer Röhrenhochspannung versorgt werden kann. Insbesondere ist die elektronische Schaltung derart mit der Kathode und der Anode verbunden, dass ein positiver Spannungsanteil der Röhrenspannung an der Anode angelegt werden kann und ein negativer Spannungsanteil der Röhrenspannung an der Kathode angelegt werden kann. Neben der elektronischen Schaltung und der Röntgenröhre kann das Röntgenröhrensystem weitere Komponenten enthalten wie zum Beispiel ein Gehäuse.

Weitere Ausführungsformen des erfindungsgemäßen Röntgenröhrensystems folgen unmittelbar aus den verschiedenen Ausgestaltungen der erfindungsgemäßen elektronischen Schaltung. Insbesondere lassen sich einzelne Merkmale und entsprechende Erläuterungen sowie Vorteile bezüglich der verschiedenen Ausführungsformen zu den erfindungsgemäßen Vorrichtungen analog auf entsprechende Ausführungsformen des erfindungsgemäßen Röntgenröhrensystems übertragen.

Gemäß einem weiteren Aspekt der Erfindung wird ein medizinisches Bildgebungssystem aufweisend ein erfindungsgemäßes Röntgenröhrensystem angegeben.

Das medizinische Bildgebungssystem kann zum Beispiel ein Röntgensystem, insbesondere ein digitales Röntgensystem, sowohl ein stationäres als auch ein mobiles System, sein, oder ein spezialisiertes Röntgengerät, wie ein Computertomographiesystem (CT-Systeme), ein Kegelstrahl-CT-System, ein Mammographiesystem, ein Dentalröntgensystem, ein Fluoroskopiesystem, ein Angiographiesystem, ein C-Bogen-System oder auch ein klassisches Röntgengerät.

Weitere Ausführungsformen des erfindungsgemäßen medizinischen Bildgebungssystems folgen unmittelbar aus den verschiedenen Ausgestaltungen des erfindungsgemäßen Röntgenröhrensystems oder der erfindungsgemäßen elektronischen Schaltung. Insbesondere lassen sich einzelne Merkmale und entsprechende Erläuterungen sowie Vorteile bezüglich der verschiedenen Ausführungsformen zu den erfindungsgemäßen Vorrichtungen analog auf entsprechende Ausführungsformen des erfindungsgemäßen medizinischen Bildgebungssystems übertragen.

Gemäß einem weiteren Aspekt der Erfindung wird ein Verfahren zum Bereitstellen einer Röhrenhochspannung einer Röntgenröhre vorgestellt. Dabei wird eine Eingangsgleichspannung abhängig von einer ersten Stellgröße in eine erste Wechselspannung gewandelt und die Eingangsgleichspannung abhängig von einer zweiten Stellgröße in eine zweite Wechselspannung gewandelt. Außerdem wird die Röhrenhochspannung abhängig von der ersten Wechselspannung und der zweiten Wechselspannung erzeugt und die Röhrenhochspannung zur Verfügung gestellt. Zudem wird eine Regelgröße bestimmt abhängig von
i) einem aus der ersten Wechselspannung resultierenden ersten Wechselstrom und einem aus der zweiten Wechselspannung resultierenden zweiten Wechselstrom und/oder
ii) einem aus der Eingangsgleichspannung resultierenden ersten Gleichstrom an der ersten Wechselrichtereinheit und einem aus der Eingangsgleichspannung resultierenden zweiten Gleichstrom an der zweiten Wechselrichtereinheit.

Zusätzlich wird die erste Stellgröße und/oder die zweite Stellgröße abhängig von der Regelgröße verändert, um die Regelgröße auf einen vorgegebenen Sollwert zu regeln.

Gemäß zumindest einer Ausführungsform des Verfahrens ist die Regelgröße abhängig von einer Differenz zwischen dem ersten Wechselstrom und dem zweiten Wechselstrom oder von einer Differenz zwischen dem ersten Gleichstrom und dem zweiten Gleichstrom oder die Regelgröße ist abhängig von einer Differenz zwischen einem ersten charakteristischen Wert des ersten Wechselstroms und einem zweiten charakteristischen Wert des zweiten Wechselstroms. Der erste charakteristische Wert entspricht einem ersten Effektivwert, einem ersten Gleichrichtwert oder einem ersten Spitzenwert und der zweite charakteristische Wert entspricht einem zweiten Effektivwert, einem zweiten Gleichrichtwert oder einem zweiten Spitzenwert.

Weitere Ausführungsformen des erfindungsgemäßen Verfahrens folgen unmittelbar aus den verschiedenen Ausgestaltungen der erfindungsgemäßen elektronischen Schaltung und umgekehrt. Insbesondere lassen sich einzelne Merkmale und entsprechende Erläuterungen sowie Vorteile bezüglich der verschiedenen Ausführungsformen zu der erfindungsgemäßen elektronischen Schaltung analog auf entsprechende Ausführungsformen des erfindungsgemäßen Verfahrens übertragen. Insbesondere ist die erfindungsgemäße elektronische Schaltung zum Durchführen eines erfindungsgemäßen Verfahrens ausgebildet oder programmiert. Insbesondere führt die erfindungsgemäße elektronische Schaltung das erfindungsgemäße Verfahren durch.

Gemäß einem weiteren Aspekt der Erfindung wird ein Verfahren zum Betreiben einer Röntgenröhre vorgestellt, wobei eins der vorgestellten Verfahren zum Bereitstellen einer Röhrenhochspannung einer Röntgenröhre durchgeführt wird und mittels der Röntgenröhre abhängig von der Röhrenhochspannung Röntgenstrahlung erzeugt wird.

Weitere Ausführungsformen des erfindungsgemäßen Verfahrens folgen unmittelbar aus den verschiedenen Ausgestaltungen der erfindungsgemäßen elektronischen Schaltung und umgekehrt. Insbesondere lassen sich einzelne Merkmale und entsprechende Erläuterungen sowie Vorteile bezüglich der verschiedenen Ausführungsformen zu der erfindungsgemäßen elektronischen Schaltung analog auf entsprechende Ausführungsformen des erfindungsgemäßen Verfahrens übertragen. Insbesondere ist die erfindungsgemäße elektronische Schaltung zum Durchführen eines erfindungsgemäßen Verfahrens ausgebildet oder programmiert. Insbesondere führt die erfindungsgemäße elektronische Schaltung das erfindungsgemäße Verfahren durch.

Weitere Merkmale und Merkmalskombinationen der Erfindung ergeben sich aus den Figuren und deren Beschreibung sowie aus den Ansprüchen. Insbesondere müssen weitere Ausführungsformen der Erfindung nicht unbedingt alle Merkmale eines der Ansprüche enthalten. Weitere Ausführungsformen der Erfindungen können Merkmale oder Merkmalskombinationen aufweisen, die nicht in den Ansprüchen genannt sind.

Die Erfindung wird im Folgenden anhand konkreter Ausführungsbeispiele und zugehöriger schematischer Zeichnungen näher erläutert. In den Figuren können gleiche oder funktionsgleiche Elemente mit denselben Bezugszeichen versehen sein. Die Beschreibung gleicher oder funktionsgleicher Elemente wird gegebenenfalls nicht notwendigerweise bezüglich verschiedener Figuren wiederholt.

Dabei zeigen
- FIG 1: ein schematisches Blockschaltbild einer beispielhaften Ausführungsform einer erfindungsgemäßen elektronischen Schaltung; und
- FIG 2: ein schematisches Blockschaltbild einer weiteren beispielhaften Ausführungsform einer erfindungsgemäßen elektronischen Schaltung; und
- FIG 3: ein schematisches Blockschaltbild eines Teils einer Regelanordnung einer weiteren beispielhaften Ausführungsform einer erfindungsgemäßen elektronischen Schaltung; und
- FIG 4: ein schematisches Blockschaltbild eines weiteren Teils einer Regelanordnung einer weiteren beispielhaften Ausführungsform einer erfindungsgemäßen elektronischen Schaltung; und
- FIG 5: ein schematisches Blockschaltbild eines weiteren Teils einer Regelanordnung einer weiteren beispielhaften Ausführungsform einer erfindungsgemäßen elektronischen Schaltung.

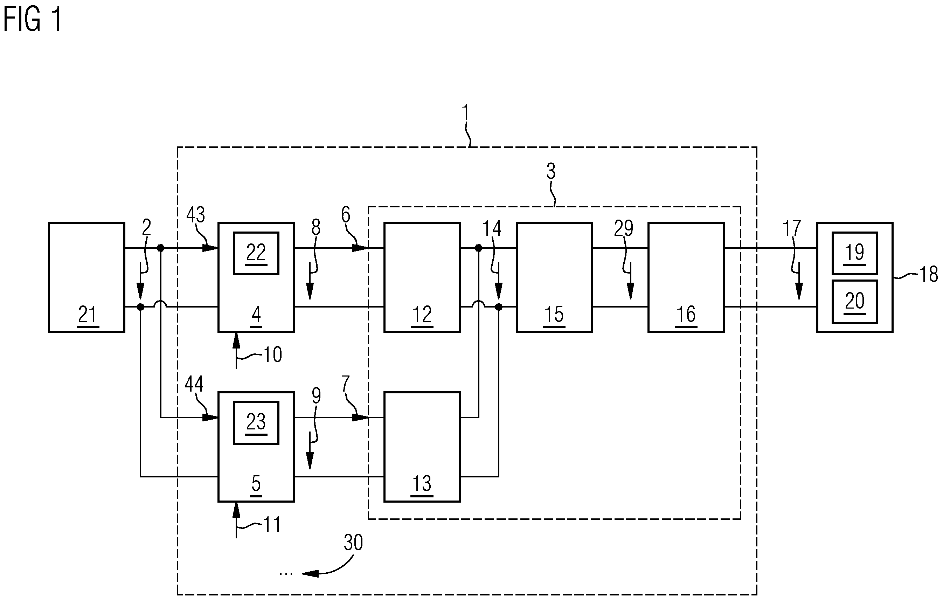

In FIG 1 ist eine beispielhafte Ausführungsform einer erfindungsgemäßen elektronischen Schaltung 1 zum Bereitstellen einer Röhrenhochspannung 17 für eine Röntgenröhre 18 gezeigt. Die elektronische Schaltung 1 weist eine erste Wechselrichtereinheit 4 auf, die dazu eingerichtet ist, eine Eingangsgleichspannung 2 zu erhalten und die Eingangsgleichspannung 2 abhängig von einer ersten Stellgröße 10 in eine erste Wechselspannung 8 zu wandeln. Außerdem weist die elektronische Schaltung 1 eine zweite Wechselrichtereinheit 5 auf, die dazu eingerichtet ist, die Eingangsgleichspannung 2 zu erhalten und die Eingangsgleichspannung 2 abhängig von einer zweiten Stellgröße 11 in eine zweite Wechselspannung 9 zu wandeln. Ebenso weist die elektronische Schaltung 1 einen weiteren Schaltungsteil 3 auf, der dazu eingerichtet ist, abhängig von der ersten Wechselspannung 8 und der zweiten Wechselspannung 9 die Röhrenhochspannung 17 zu erzeugen und die Röhrenhochspannung 17 ausgangsseitig zur Verfügung zu stellen. Die elektronische Schaltung 1 weist außerdem eine Regelanordnung 24 auf, die dazu eingerichtet ist, eine Regelgröße 25 zu bestimmen und die erste Stellgröße 10 und/oder die zweite Stellgröße 11 abhängig von der Regelgröße 25 zu verändern, um die Regelgröße 25 auf einen vorgegebenen Sollwert 26 zu regeln. Die Regelanordnung 24 ist dazu eingerichtet, die Regelgröße 25 zu bestimmen abhängig von
i) einem aus der ersten Wechselspannung 8 resultierenden ersten Wechselstrom 6 und einem aus der zweiten Wechselspannung 9 resultierenden zweiten Wechselstrom 7 und/oder
ii) einem aus der Eingangsgleichspannung 2 resultierenden ersten Gleichstrom 43 an der ersten Wechselrichtereinheit 4 und einem aus der Eingangsgleichspannung 2 resultierenden zweiten Gleichstrom 44 an der zweiten Wechselrichtereinheit 5.

FIG 1 zeigt ebenso schematisch die Röntgenröhre 18, die eine Anode 19 und eine Kathode 20 enthält. Die Röhrenhochspannung 17 wird durch die elektronische Schaltung 1 ausgangsseitig zur Verfügung gestellt und kann zur Erzeugung von Röntgenstrahlung mit einem positiven elektrischen Potential an die Anode 19 und mit einem negativen elektrischen Potential an die Kathode 20 gekoppelt werden. Eine Kopplung kann dabei insbesondere durch eine elektrisch leitfähige Verbindung hergestellt werden. Daneben wird in FIG 1 eine Gleichspannungsquelle 21 gezeigt, die die Eingangsgleichspannung 2 zur Verfügung stellen kann, insbesondere der elektronischen Schaltung 1 zur Verfügung stellen kann.

Die Röntgenröhre 18 ist nicht Teil der erfindungsgemäßen elektronischen Schaltung 1. Jedoch bilden die erfindungsgemäße elektronische Schaltung 1 und die Röntgenröhre 18 beispielsweise eine erfindungsgemäßes Röntgenröhrensystem. Das Röntgenröhrensystem ist insbesondere Teil eines röntgenbasierten medizinischen Bildgebungssystems oder für ein röntgenbasiertes medizinisches Bildgebungssystem vorgesehen. Die Gleichspannungsquelle 21 ist nicht notwendigerweise Teil der erfindungsgemäßen elektronischen Schaltung 1. Die Gleichspannungsquelle 21 kann in manchen Ausführungsformen aber Teil der erfindungsgemäßen elektronischen Schaltung 1 oder des erfindungsgemäßen Röntgenröhrensystems sein.

Die erste Wechselrichtereinheit 4 kann einen ersten Wechselrichter 22 enthalten. Ebenso kann die zweite Wechselrichtereinheit 5 einen zweiten Wechselrichter 23 enthalten. Sowohl der erste Wechselrichter 22 als auch der zweite Wechselrichter 23 können dabei beispielsweise als PWM-Wechselrichter ausgeführt werden. Der erste Wechselrichter 22 kann insbesondere die erste Wechselspannung 8 ausgangsseitig zur Verfügung stellen, die abhängig ist von der ersten Stellgröße 10. Die erste Stellgröße 10 kann dabei beispielsweise einem ersten Aussteuergrad des ersten Wechselrichters 22 entsprechen. Der zweite Wechselrichter 23 kann insbesondere die zweite Wechselspannung 8 ausgangsseitig zur Verfügung stellen, die abhängig ist von der zweiten Stellgröße 10. Die zweite Stellgröße 10 kann dabei beispielsweise einem zweiten Aussteuergrad des zweiten Wechselrichters 22 entsprechen.

Sowohl die erste Wechselrichtereinheit 4, als auch die Wechselrichtereinheit 5 können eingangsseitig die Eingangsgleichspannung 2 erhalten. Die Eingangsgleichspannung 2 kann von einer Gleichspannungsquelle 21 zur Verfügung gestellt werden.

Der weitere Schaltungsteil 3 ist in FIG 1 beispielhaft dargestellt aufweisend einen ersten Resonanzkreis 12, einen zweiten Resonanzkreis 13, einen Spannungstransformator 15 und einen Gleichrichter 16. Darüber hinaus kann der weitere Schaltungsteil 3 einen ersten und einen zweiten Knotenpunkt aufweisen. Der erste und der zweite Knotenpunkt stellen beispielsweise eine elektrisch leitende Verbindung dar und können jeweils einen Ausgang des ersten Resonanzkreises 12 mit jeweils einem Ausgang des zweiten Resonanzkreises 13 koppeln. Zwischen dem ersten Knotenpunkt und dem zweiten Knotenpunkt kann eine Primärwechselspannung 14 anliegen. Diese Primärwechselspannung 14 kann insbesondere als eine Überlagerung oder Kombination einer Ausgangsspannung des ersten Resonanzkreises 12 und einer Ausgangsspannung des zweiten Resonanzkreises 13 betrachtet werden. Der Spannungstransformator 15 kann abhängig von einem Übersetzungsverhältnis die Primärwechselspannung 14 in eine Sekundärwechselspannung 29 wandeln und diese ausgangsseitig zur Verfügung stellen. Der Gleichrichter 16 kann diese Sekundärwechselspannung 29 eingangsseitig erhalten und diese in die Röhrenhochspannung 17 wandeln, die der Röntgenröhre 18 zur Verfügung gestellt werden kann.

Insbesondere sind auch abweichende Schaltungen des Weiteren Schaltungsteils 3 denkbar, zum Beispiel beinhaltend einen weiteren Spannungstransformator. In diesem Fall kann der Ausgang des ersten Resonanzkreises 12 mit dem Spannungstransformator 15 verbunden sein und der Ausgang des zweiten Resonanzkreises 13 mit dem weiteren Spannungstransformator verbunden sein. Darüber hinaus kann der weitere Schaltungsteil 3 einen dritten und einen vierten Knotenpunkt aufweisen. Der dritte Knotenpunkt kann einen ersten Ausgang des Spannungstransformators 15 mit einem ersten Ausgang des weiteren Spannungstransformators verbinden und der vierte Knotenpunkt kann einen zweiten Ausgang des Spannungstransformators 15 mit einem zweiten Ausgang des weiteren Spannungstransformators verbinden. Zwischen dem dritten und dem vierten Knotenpunkt kann dann eine weitere Sekundärwechselspannung anliegen.

Ebenso kann der weitere Schaltungsteil 3 einen weiteren Gleichrichter enthalten. In diesem Fall kann ein Ausgang des weiteren Spannungstransformators mit einem Eingang des weiteren Gleichrichters verbunden sein. Ein fünfter Knotenpunkt kann einen ersten Ausgang des Gleichrichters 16 mit einem ersten Ausgang des weiteren Gleichrichters verbinden und ein sechster Knotenpunkt einen zweiten Ausgang des Gleichrichter 16 mit einem zweiten Ausgang des weiteren Gleichrichters. Zwischen dem fünften und dem sechsten Knotenpunkt kann dann die Röhrenhochspannung 17 anliegen.

Die an einem Ausgang der ersten Wechselrichtereinheit 4 anliegende erste Wechselspannung 8 kann insbesondere aufgrund einer anliegenden Beschaltung, insbesondere einer Beschaltung durch den weiteren Schaltungsteil 3, den ersten Wechselstrom 6 hervorrufen. Ebenso kann die an einem Ausgang der zweiten Wechselrichtereinheit 5 anliegende zweite Wechselspannung 9 insbesondere aufgrund einer dort anliegenden Beschaltung, insbesondere der Beschaltung durch den weiteren Schaltungsteil 3, den zweiten Wechselstrom 7 hervorrufen.

Die an einem Eingang der ersten Wechselrichtereinheit 4 anliegende Eingangsgleichspannung 2 kann an der ersten Wechselrichtereinheit 4 den ersten Gleichstrom 43 hervorrufen. Ebenso kann die an einem Eingang der zweiten Wechselrichtereinheit 5 anliegende Eingangsgleichspannung 2 an der zweiten Wechselrichtereinheit 5 den zweiten Gleichstrom 44 hervorrufen.

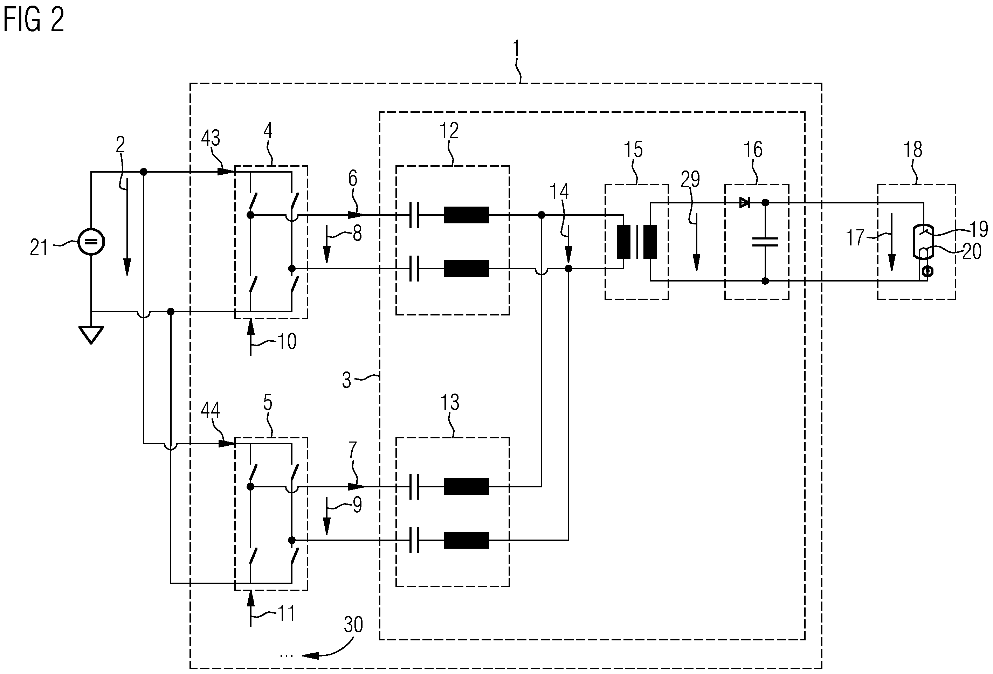

Eine weitere beispielhafte Ausführungsform der elektronischen Schaltung 1 ist im Blockschaltdiagramm in FIG 2 gezeigt. Das in FIG 2 gezeigte Ausführungsbeispiel kann analog alle Merkmale einer der bezüglich FIG 1 beschriebenen und/oder in FIG 1 gezeigten Ausführungsform aufweisen, wenn nicht abweichend dargestellt oder erwähnt.

FIG 2 zeigt beispielhaft an einem Ausgang der Gleichspannungsquelle 21 ein Massepotential. Insbesondere kann ein weiterer Ausgang der Gleichspannungsquelle 21 ein elektrisch positives Potential aufweisen. Zwischen dem Ausgang der Gleichspannungsquelle 21 und dem weiteren Ausgang der Gleichspannungsquelle 21 kann die Eingangsgleichspannung 2 anliegen.

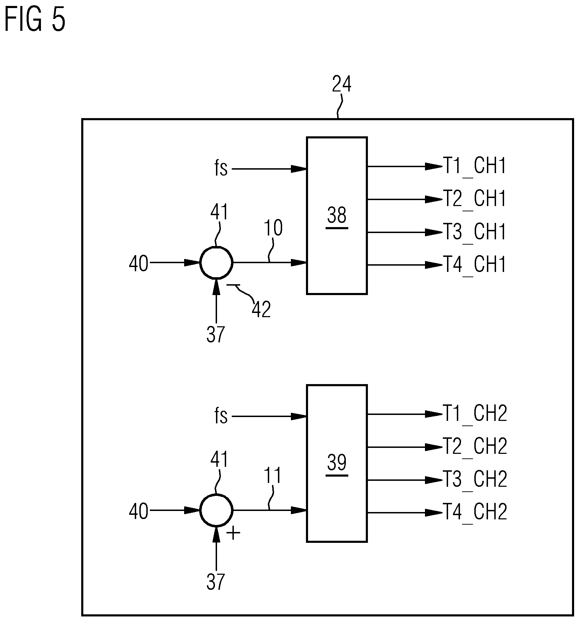

Die erste Wechselrichtereinheit 4 ist in FIG 2 beispielhaft mit vier Schaltern dargestellt. Diese Ausführungsform kann auch als Vollbrücke bezeichnet werden. Die vier Schalter können beispielhaft von vier Steuerleitungen T1_CH1, T2_CH1, T3_CH1, T4_CH1 einer ersten Steuersignalerzeugung 38 angesteuert werden. Eine beispielhafte Möglichkeit zur Realisierung der ersten Steuersignalerzeugung 38 ist in FIG 5 dargestellt. Dabei kann jeweils eine Steuerleitung T1_CH1, T2_CH1, T3_CH1, T4_CH1 einer Ansteuerung eines Schalters entsprechen. Zum Beispiel kann eine erste Steuerleitung T1_CH1 einen ersten Schalter ansteuern, eine zweite Steuerleitung T2_CH1 einen zweiten Schalter usw.

Ebenso ist die zweite Wechselrichtereinheit 5 ist in FIG 2 beispielhaft mit vier weiteren Schaltern dargestellt. Diese Ausführungsform kann auch als Vollbrücke bezeichnet werden. Die vier weiteren Schalter können beispielhaft von vier weiteren Steuerleitungen T1_CH2, T2_CH2, T3_CH2, T4_CH2 einer zweiten Steuersignalerzeugung 39 angesteuert werden. Eine beispielhafte Möglichkeit zur Realisierung der zweiten Steuersignalerzeugung 39 ist ebenfalls in FIG 5 dargestellt. Dabei kann jeweils eine weitere Steuerleitung T1_CH2, T2_CH2, T3_CH2, T4_CH2 einer weiteren Ansteuerung eines weiteren Schalters entsprechen. Zum Beispiel kann eine erste weitere Steuerleitung T1_CH2 einen ersten weiteren Schalter ansteuern, eine zweite weitere Steuerleitung T2_CH2 einen zweiten weiteren Schalter usw.

Die erste Steuersignalerzeugung 38 und die zweite Steuersignalerzeugung 39 können ebenso als Pulsmaschinen bezeichnet werden.

Der erste Resonanzkreis 12 kann zum Beispiel eine Serienschaltung eines Kondensators und einer Spule in einem ersten Signalpfad aufweisen und eine weitere Serienschaltung eines weiteren Kondensators und einer weiteren Spule in einem zweiten Signalpfad. Der zweite Resonanzkreis 13 kann beispielsweise analog aufgebaut sein. Alternativ kann sich der erste Resonanzkreis 12 von dem zweiten Resonanzkreis 13 unterscheiden, insbesondere in Bezug auf Typ, Größenordnung, Toleranz oder Beschaltung von Bauelementen.

Der Gleichrichter 16 ist in der Ausführungsform in FIG 2 beispielhaft aufweisend eine Diode und einen Kondensator dargestellt. Ebenso sind weitere Bauelemente denkbar oder gegebenenfalls vorteilhaft.

Die dargestellten Ausführungsformen der elektronischen Schaltung 1 in FIG 1 und FIG 2 sind ebenso erweiterbar zum Beispiel durch wenigstens eine weitere Wechselrichtereinheit 30 (in FIG 1 und FIG 2 durch drei Punkte dargestellt). Die bereits dargestellten Eigenschaften der ersten Wechselrichtereinheit 4 und der zweiten Wechselrichtereinheit 5 können ebenfalls Gültigkeit für die wenigstens eine weitere Wechselrichtereinheit 30 haben. Insbesondere kann die Eingangsgleichspannung 2 mit einem Eingang der wenigstens einen weiteren Wechselrichtereinheit 30 verbunden sein. Jede der wenigstens einen weiteren Wechselrichtereinheit 30 kann jeweils abhängig sein von wenigstens einer weiteren Stellgröße 33. Dabei kann sich jede der wenigstens einen weiteren Stellgröße von jeder anderen der wenigstens einen weiteren Stellgröße unterscheiden.

Des Weiteren kann der weitere Schaltungsteil 3 wenigstens einen weiteren Resonanzkreis enthalten, der zum Beispiel mit einem Ausgang der wenigstens einen weiteren Wechselrichtereinheit 30 verbunden ist. Der wenigstens eine weitere Resonanzkreis kann analoge Eigenschaften des ersten Resonanzkreises oder des zweiten Resonanzkreises aufweisen. Ein erster Ausgang des wenigstens einen weiteren Resonanzkreises kann ebenso mit dem ersten Knotenpunkt verbunden sein, sowie ein zweiter Ausgang des wenigstens einen weiteren Resonanzkreises mit dem zweiten Knotenpunkt. In diesen beiden Knotenpunkten kann zum Beispiel eine Leistung aller dort angeschlossenen Kombinationen aus Wechselrichtereinheit und Resonanzkreis zusammengeführt werden und von dort aus über darauffolgende Bauelemente zur Röntgenröhre 18 geführt werden.

Jeder der wenigstens einen weiteren Wechselrichtereinheit 30 kann ausgangsseitig jeweils wenigstens eine weitere Wechselspannung aufweisen. Diese wenigstens eine weitere Wechselspannung kann aufgrund wenigstens einer daran angeschlossenen Beschaltung wenigstens einen weiteren Wechselstrom hervorrufen. Die Regelgröße 25 kann neben dem ersten Wechselstrom 6 und dem zweiten Wechselstrom 7 auch von jedem einzelnen des wenigstens einen weiteren Wechselstroms abhängig sein.

Jeder der wenigstens einen weiteren Wechselrichtereinheit 30 kann eingangsseitig jeweils wenigstens einen weiteren Gleichstrom aufweisen, der durch die angelegte Eingangsgleichspannung resultieren kann. Die Regelgröße 25 kann neben dem ersten Gleichstrom 43 und dem zweiten Gleichstrom 44 auch von jedem einzelnen des wenigstens einen weiteren Gleichstroms abhängig sein.

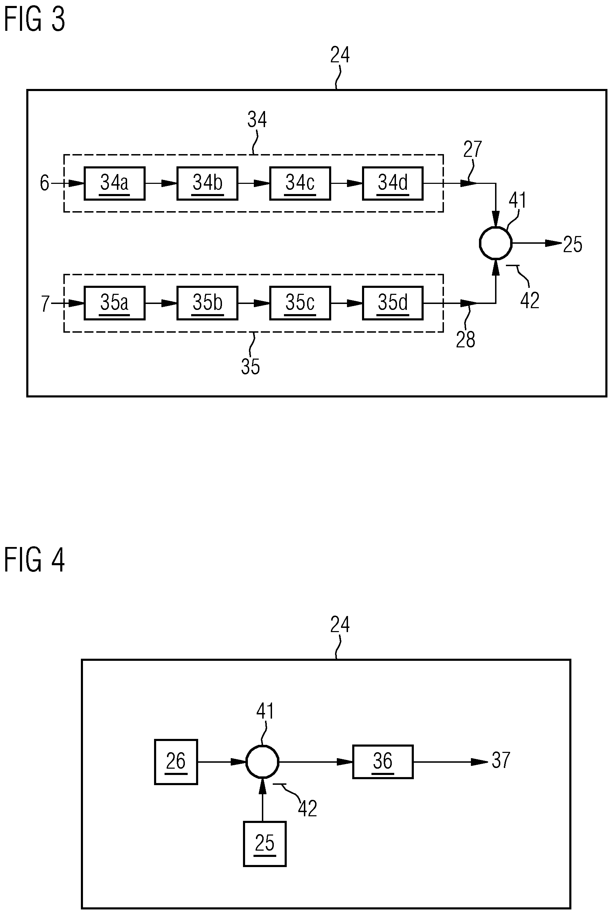

FIG 3 zeigt beispielhaft einen Teil einer Regelanordnung 24 einer weiteren beispielhaften Ausführungsform einer erfindungsgemäßen elektronischen Schaltung 1. Dabei kann der erste Wechselstrom 6 zunächst mit einer ersten Filtereinheit 34 verbunden sein. Die erste Filtereinheit 34 kann zum Beispiel einen ersten Analog-Digital-Konverter 34a enthalten, der aus dem analogen ersten Wechselstrom 6 ein erstes digitales Signal erstellen kann. Daraufhin kann das erste digitale Signal in einer Filterkomponente 34b quadriert werden. Anschließend kann die erste Filtereinheit 34 zum Beispiel ein Tiefpassfilter 34c enthalten. Abschließend kann aus einem Ausgangssignal des Tiefpassfilters 34c eine Quadratwurzel innerhalb einer weiteren Filterkomponente 34d gezogen werden. Ein Ausgangssignal der ersten Filtereinheit 34 kann zum Beispiel ein erster charakteristischer Wert 27 sein. Insbesondere kann die erste Filtereinheit 34 ein RMS-Filter (root mean square Filter) in der dargestellten oder in einer abweichenden Form aufweisen.

Gleiches gilt für die zweite Filtereinheit 35. Ausgangsseitig kann die zweite Filtereinheit 35 einen zweiten charakteristischen Wert 28 zur Verfügung stellen. Die Regelanordnung 24 kann darüber hinaus einen Inverter 42 und einen Addierer 41 enthalten. Zum Beispiel kann mit dem Addierer 41 der erste charakteristische Wert 27 zu dem invertierten zweiten charakteristischen Wert 28 hinzugezählt werden. Ausgangsseitig kann der Addierer 41 die Regelgröße 25 zur Verfügung stellen.

Der erste charakteristische Wert kann einem ersten Effektivwert, einem ersten Gleichrichtwert oder einem ersten Spitzenwert entsprechen und der zweite charakteristische Wert kann einem zweiten Effektivwert, einem zweiten Gleichrichtwert oder einem zweiten Spitzenwert entsprechen. Die Werte für einen Gleichrichtwert und/oder für einen Spitzenwert können dabei jeweils innerhalb einer Schaltperiode ermittelt werden.

In FIG 4 wird ein weiterer Teil einer Regelanordnung 24 einer weiteren beispielhaften Ausführungsform einer erfindungsgemäßen elektronischen Schaltung schematisch gezeigt. Beispielsweise kann der in FIG 4 gezeigte Teil mit dem in FIG 3 gezeigten Teil kombiniert werden. Es kann sich also insbesondere um Teile derselben Regelanordnung 24 handeln.

Der vorgegebene Sollwert 26 kann mit einem Eingang eines weiteren Addierers 41 verbunden sein. Die Regelgröße 25 kann zunächst mit einem weiteren Inverter 42 verbunden sein. Ein Ausgangssignal des weiteren Inverters 42 kann mit einem weiteren Eingang des Weiteren Addierers 41 verbunden sein. Ein Ausgang des Weiteren Addierers 41 kann mit einem Regler 36 verbunden sein. Der Regler 36 kann insbesondere einen PI-Regler oder einen PID-Regler enthalten. An einem Ausgang des Reglers 36 kann eine Reglerstellgröße 37 zur Verfügung gestellt werden.

FIG 5 zeigt einen weiteren Teil einer Regelanordnung 24 einer weiteren beispielhaften Ausführungsform einer erfindungsgemäßen elektronischen Schaltung schematisch. Beispielsweise kann der in FIG 5 gezeigte Teil mit dem in FIG 3 gezeigten Teil und/oder dem in FIG 4 gezeigten Teil kombiniert werden. Es kann sich also insbesondere um Teile derselben Regelanordnung 24 handeln.

Ein vorgegebener Initialwert 40 kann mit einem ersten Eingang eines ersten anderen Addierers 41 verbunden sein. Die Reglerstellgröße 37 kann mit einem Eingang eines anderen Inverters 42 verbunden sein und ein Ausgang des anderen Inverters 42 mit einem weiteren Eingang des ersten anderen Addierers 41. Der erste andere Addierer kann an einem Ausgang die erste Stellgröße zur Verfügung stellen. In dieser Ausführungsform ist ebenso die erste Steuersignalerzeugung 38 gezeigt, die an einem Eingang die erste Stellgröße 10 erhalten kann und an einem weiteren Eingang eine Schaltfrequenz fs. Die Schaltfrequenz fs kann insbesondere für alle Wechselrichtereinheiten 4, 5, 30 zur Vermeidung von Kreisströmen gleich gewählt werden. Die erste Steuersignalerzeugung 38 kann wie oben beschrieben beispielhaft die vier Steuerleitungen T1_CH1, T2_CH1, T3_CH1, T4_CH1 zur Verfügung stellen, die die vier Schalter der ersten Wechselrichtereinheit 4 ansteuern können.

Ebenso zeigt FIG 5 zeigt die zweite Steuersignalerzeugung 39. Der vorgegebener Initialwert 40 kann mit einem ersten Eingang eines zweiten anderen Addierers 41 verbunden sein und die Reglerstellgröße 37 mit einem weiteren Eingang des zweiten anderen Addierers 41 verbunden sein. Im Gegensatz zu der ersten Steuersignalerzeugung 38 ist in diesem Fall keine Inverterstufe enthalten. Ebenso kann eine Ausführungsform mit einer vertauschten Konfiguration vorteilhaft sein, also eine Inverterstufe an der zweiten Steuersignalerzeugung 39 und keine Inverterstufe an der ersten Steuersignalerzeugung 38. Der zweite andere Addierer kann an einem Ausgang die zweite Stellgröße zur Verfügung stellen. Die zweite Steuersignalerzeugung 39 kann an einem Eingang die zweite Stellgröße 11 erhalten und an einem weiteren Eingang die Schaltfrequenz fs. Die zweite Steuersignalerzeugung 39 kann wie oben beschrieben beispielhaft die vier weiteren Steuerleitungen T1_CH2, T2_CH2, T3_CH2, T4_CH2 zur Verfügung stellen, die die vier weiteren Schalter der zweiten Wechselrichtereinheit 5 ansteuern können.

Die Regelanordnung 24 kann ebenso gleich geartete Schaltungen enthalten, die eine Ansteuerung der wenigsten einen weiteren Wechselrichtereinheit 30 ermöglichen.

Unabhängig vom grammatikalischen Geschlecht eines bestimmten Begriffes sind Personen mit männlicher, weiblicher oder anderer Geschlechteridentität mit umfasst.

## Patentansprüche

1. Elektronische Schaltung (1) zum Bereitstellen einer Röhrenhochspannung (17) für eine Röntgenröhre (18), die elektronische Schaltung (1) aufweisend
- eine erste Wechselrichtereinheit (4), die dazu eingerichtet ist, eine Eingangsgleichspannung (2) zu erhalten und die Eingangsgleichspannung (2) abhängig von einer ersten Stellgröße (10) in eine erste Wechselspannung (8) zu wandeln; und
- eine zweite Wechselrichtereinheit (5), die dazu eingerichtet ist, die Eingangsgleichspannung (2) zu erhalten und die Eingangsgleichspannung (2) abhängig von einer zweiten Stellgröße (11) in eine zweite Wechselspannung (9) zu wandeln; und
- einen weiteren Schaltungsteil (3), der dazu eingerichtet ist, abhängig von der ersten Wechselspannung (8) und der zweiten Wechselspannung (9) die Röhrenhochspannung (17) zu erzeugen und die Röhrenhochspannung (17) ausgangsseitig zur Verfügung zu stellen; und
- eine Regelanordnung (24), die dazu eingerichtet ist, eine Regelgröße (25) zu bestimmen und die erste Stellgröße (10) und/oder die zweite Stellgröße (11) abhängig von der Regelgröße (25) zu verändern, um die Regelgröße (25) auf einen vorgegebenen Sollwert (26) zu regeln, wobei die Regelanordnung (24) dazu eingerichtet ist, die Regelgröße (25) zu bestimmen abhängig von
i) einem aus der ersten Wechselspannung (8) resultierenden ersten Wechselstrom (6) und einem aus der zweiten Wechselspannung (9) resultierenden zweiten Wechselstrom (7) und/oder
ii) einem aus der Eingangsgleichspannung (2) resultierenden ersten Gleichstrom (43) an der ersten Wechselrichtereinheit (4) und einem aus der Eingangsgleichspannung (2) resultierenden zweiten Gleichstrom (44) an der zweiten Wechselrichtereinheit (5).

2. Elektronische Schaltung (1) gemäß Anspruch 1, wobei die Regelgröße (25) von einer Differenz zwischen dem ersten Wechselstrom (6) und dem zweiten Wechselstrom (7) und/oder einer Differenz zwischen dem ersten Gleichstrom (43) und dem zweiten Gleichstrom (44) abhängt.

3. Elektronische Schaltung (1) gemäß Anspruch 1, wobei
- die Regelanordnung (24) dazu eingerichtet ist, aus dem ersten Wechselstrom (6) einen ersten charakteristischen Wert (27) und aus dem zweiten Wechselstrom (7) einen zweiten charakteristischen Wert (28) zu bestimmen;
- die Regelgröße (25) abhängig ist von dem ersten charakteristischen Wert (27) und dem zweiten charakteristischen Wert (28) oder die Regelgröße (25) abhängig ist von einer Differenz zwischen dem ersten charakteristischen Wert (27) und dem zweiten charakteristischen Wert (28);
- der erste charakteristische Wert einem ersten Effektivwert, einem ersten Gleichrichtwert oder einem ersten Spitzenwert entspricht; und
- der zweite charakteristische Wert einem zweiten Effektivwert, einem zweiten Gleichrichtwert oder einem zweiten Spitzenwert entspricht.

4. Elektronische Schaltung (1) gemäß Anspruch 3, wobei die Regelanordnung (24) dazu eingerichtet ist, abhängig von dem ersten charakteristischen Wert (27) und dem zweiten charakteristischen Wert (28) einen größten charakteristischen Wert zu ermitteln, und
- die erste Stellgröße (10) abhängig von der Regelgröße (25) zu verändern, wenn der erste charakteristische Wert (27) geringer als der größte charakteristische Wert ist; und
- die zweite Stellgröße (11) abhängig von der Regelgröße (25) zu verändern, wenn der zweite charakteristische Wert (28) geringer als der größte charakteristische Wert ist.

5. Elektronische Schaltung (1) gemäß einem der vorherigen Ansprüche 1 bis 3, wobei die Regelanordnung (24) dazu eingerichtet ist, abhängig von der Regelgröße (25) und dem vorgegebenen Sollwert (26) eine Reglerstellgröße (37) zu ermitteln, und
- die erste Stellgröße (10) von einer Differenz eines vorgegebenen Initialwerts (40) und der Reglerstellgröße (37) abhängt; und/oder
- die zweite Stellgröße (11) von einer Summe des vorgegebenen Initialwerts (40) und der Reglerstellgröße (37) abhängt.

6. Elektronische Schaltung (1) gemäß einem der vorherigen Ansprüche, wobei der weitere Schaltungsteil (3) einen Spannungstransformator (15) enthält, der dazu eingerichtet ist, primärseitig eine aus der ersten Wechselspannung (8) und/oder der zweiten Wechselspannung (9) resultierende Primärwechselspannung (14) zu erhalten und die Primärwechselspannung (14) in eine Sekundärwechselspannung (29) zu wandeln, wobei die Röhrenhochspannung (17) abhängig von der Sekundärwechselspannung (29) ist.

7. Elektronische Schaltung (1) gemäß Anspruch 6, wobei der weitere Schaltungsteil (3) einen Gleichrichter (16) enthält, der dazu eingerichtet ist, die Sekundärwechselspannung (29) in die Röhrenhochspannung (17) zu wandeln.

8. Elektronische Schaltung (1) gemäß einem der vorherigen Ansprüche, wobei
- der weitere Schaltungsteil (3) einen ersten Resonanzkreis (12) enthält, der eingangsseitig mit einem Ausgang der ersten Wechselrichtereinheit (4) und ausgangsseitig mit einem Ausgang des Weiteren Schaltungsteils (3), der zum Bereitstellen der Röhrenhochspannung (17) eingerichtet ist, gekoppelt ist; und
- der weitere Schaltungsteil (3) einen zweiten Resonanzkreis (13) enthält, der eingangsseitig mit einem Ausgang der zweiten Wechselrichtereinheit (5) und ausgangsseitig mit dem Ausgang des Weiteren Schaltungsteils (3), der zum Bereitstellen der Röhrenhochspannung (17) eingerichtet ist, gekoppelt ist.

9. Elektronische Schaltung (1) gemäß Anspruch 8, wobei ein Ausgang des ersten Resonanzkreises (12) und ein Ausgang des zweiten Resonanzkreises (13) miteinander gekoppelt sind.

10. Elektronische Schaltung (1) gemäß einem der vorherigen Ansprüche, wobei die elektronische Schaltung (1) wenigstens eine weitere Wechselrichtereinheit (30) enthält, wobei
- jede weitere Wechselrichtereinheit (30) der wenigstens einen weiteren Wechselrichtereinheit (30) dazu eingerichtet ist, die Eingangsgleichspannung (2) zu erhalten und die Eingangsgleichspannung (2) abhängig von einer jeweiligen weiteren Stellgröße (33) in eine jeweilige weitere Wechselspannung (31) zu wandeln;
- der weitere Schaltungsteil (3) dazu eingerichtet ist, die Röhrenhochspannung (17) abhängig von den weiteren Wechselspannungen (31) zu erzeugen;
- die Regelanordnung (24) dazu eingerichtet ist, die Regelgröße (25) zu bestimmen abhängig von
i) dem ersten Wechselstrom (6), dem zweiten Wechselstrom (7) und einem jeweiligen aus den weiteren Wechselspannungen (31) resultierenden weiteren Wechselstrom (32) und/oder
ii) dem ersten Gleichstrom (43), dem zweiten Gleichstrom (44) und einem jeweiligen aus der Eingangsgleichspannung (2) resultierenden weiteren Gleichstrom an jeder weiteren Wechselrichtereinheit (30) der wenigstens einen weiteren Wechselrichtereinheit (30).

11. Röntgenröhrensystem, aufweisend die elektronische Schaltung (1) nach einem der vorherigen Ansprüche und die Röntgenröhre (18).

12. Medizinisches Bildgebungssystem, aufweisend das Röntgenröhrensystem nach Anspruch 11.

13. Verfahren zum Bereitstellen einer Röhrenhochspannung (17) für eine Röntgenröhre (18), wobei:
- eine Eingangsgleichspannung (2) abhängig von einer ersten Stellgröße (10) in eine erste Wechselspannung (8) gewandelt wird;
- die Eingangsgleichspannung (2) abhängig von einer zweiten Stellgröße (11) in eine zweite Wechselspannung (9) gewandelt wird;
- die Röhrenhochspannung (17) abhängig von der ersten Wechselspannung (8) und der zweiten Wechselspannung (9) erzeugt und zur Verfügung gestellt wird;
- eine Regelgröße (25) bestimmt wird abhängig von
i) einem aus der ersten Wechselspannung (8) resultierenden ersten Wechselstrom (6) und einem aus der zweiten Wechselspannung (9) resultierenden zweiten Wechselstrom (7) und/oder
ii) einem aus der Eingangsgleichspannung (2) resultierenden ersten Gleichstrom (43) an der ersten Wechselrichtereinheit (4) und einem aus der Eingangsgleichspannung (2) resultierenden zweiten Gleichstrom (43) an der zweiten Wechselrichtereinheit (4); und
- die erste Stellgröße (10) und/oder die zweite Stellgröße (11) abhängig von der Regelgröße (25) verändert wird, um die Regelgröße (25) auf einen vorgegebenen Sollwert (26) zu regeln.

14. Verfahren nach Anspruch 13, wobei die Regelgröße (25) abhängig ist von einer Differenz zwischen dem ersten Wechselstrom (6) und dem zweiten Wechselstrom (7) und/oder von einer Differenz zwischen dem ersten Gleichstrom (43) und dem zweiten Gleichstrom (44) oder die Regelgröße (25) abhängig ist von einer Differenz zwischen einem ersten charakteristischen Wert (27) des ersten Wechselstroms (6) und einem zweiten charakteristischen Wert (28) des zweiten Wechselstroms (7), wobei
- der erste charakteristische Wert einem ersten Effektivwert, einem ersten Gleichrichtwert oder einem ersten Spitzenwert entspricht; und
- der zweite charakteristische Wert einem zweiten Effektivwert, einem zweiten Gleichrichtwert oder einem zweiten Spitzenwert entspricht.

15. Verfahren zum Betreiben einer Röntgenröhre (18), wobei das Verfahren nach einem der Ansprüche 13 oder 14 durchgeführt wird und mittels der Röntgenröhre (18) abhängig von der Röhrenhochspannung (17) Röntgenstrahlung erzeugt wird.
